Europäisches Patentamt

European Patent Office

Office européen des brevets

(11) Numéro de publication: **0 527 216 B1**

## (12) FASCICULE DE BREVET EUROPEEN

(45) Date de publication du fascicule du brevet: **08.11.95**  (51) Int. Cl.⁶: **A61B  5/087**, G10D 7/02

(21) Numéro de dépôt: **92905373.4**

(22) Date de dépôt: **28.02.92**

(86) Numéro de dépôt internationale :
**PCT/CH92/00040**

(87) Numéro de publication internationale :
**WO 92/15246 (17.09.92 92/24)**

(54) **APPAREIL MEDICAL.**

(30) Priorité: **28.02.91 GB 9104201**

(43) Date de publication de la demande:
**17.02.93 Bulletin  93/07**

(45) Mention de la délivrance du brevet:
**08.11.95 Bulletin  95/45**

(84) Etats contractants désignés:
**AT BE CH DE DK ES FR GB GR IT LI LU MC NL SE**

(56) Documents cités:
| | |
|---|---|
| **EP-A- 0 178 042** | **DE-A- 2 948 863** |
| **DE-A- 3 701 171** | **DE-U- 8 520 940** |
| **FR-A- 2 196 818** | **US-A- 4 232 683** |
| **US-A- 4 241 739** | **US-A- 4 744 953** |

(73) Titulaire: **ISORAW S.A.**
**Chemin de la Traverse 12**
**CH-1170 Aubonne (CH)**

(72) Inventeur: **KRAEMER, Richard**
**Schiferliweg 14**
**CH-3010 Berne (CH)**
Inventeur: **SCHIBLER, Andreas**
**Schönbergweg 12**
**CH-3006 Berne (CH)**

(74) Mandataire: **Ganguillet, Cyril et al**
**ABREMA**
**Agence Brevets & Marques**
**Ganguillet & Humphrey**
**Rue Centrale 5**
**C.P. 2065**
**CH-1002 Lausanne (CH)**

Rank Xerox (UK) Business Services
(3. 10/3.09/3.3.3)

## Description

La présente invention concerne un appareil de mesure du débit/volume d'air pulmonaire expiré par une personne.

Les courbes débit-volume, tests conventionnels de la fonction pulmonaire, sont abondamment utilisées pour mesurer la limitation du débit et, par conséquent, le degré d'obstruction bronchique des voies respiratoires fines chez les enfants et adultes atteints de maladies pulmonaires.

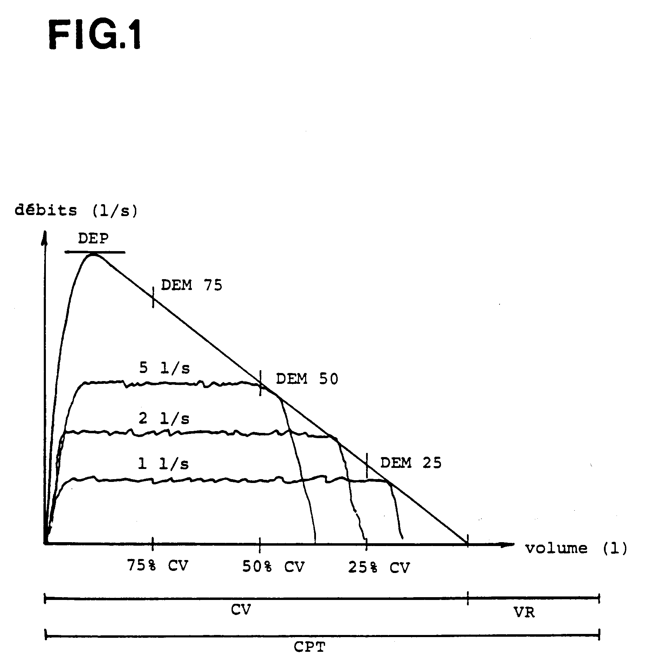

Sur la figure 1 du dessin annexé, la courbe supérieure est une courbe débit-volume obtenue en demandant au sujet d'inspirer au maximum jusqu'à ce que la capacité pulmonaire totale (CPT) soit atteinte, puis d'expirer aussi rapidement et aussi fort que possible. L'abscisse indique les variations de volume, mesurées à partir de la capacité pulmonaire totale (CPT) jusqu'au volume résiduel (VR), la différence entre les deux étant définie comme la capacité vitale (CV). L'ordonnée indique les variations de débit. Dans la première partie de la courbe, un débit élevé, nommé débit expiratoire de pointe (DEP) ou "peak flow", est atteint. Par la suite, le volume est déplacé et, chez un sujet sain, le débit diminue de façon linéaire, depuis le débit expiratoire de pointe (DEP) jusqu'au volume résiduel (VR). Les débits expiratoires maximaux à 75, 50 et 25% de la capacité vitale, définis respectivement comme DEM75, DEM50 et DEM25, sont des débits standardisés quant à un volume pulmonaire bien défini.

Les maladies spécifiques concernées dans ce domaine comprennent la bronchite obstructive et l'asthme chez les enfants, l'asthme bronchique, la mucoviscidose et les broncho-pneumopathies chroniques obstructives (BPCO) chez les adultes. Toutes ces maladies sont caractérisées par une diminution de la performance du système respiratoire, conduisant à une obstruction bronchique qui progresse avec l'âge. Fonctionnellement, l'obstruction bronchique est caractérisée par une limitation du débit d'air. Il y a grand intérêt à déterminer l'amplitude de cette diminution, afin d'obtenir une information objective quant au degré d'atteinte fonctionnelle pulmonaire et à la stabilité de la maladie. Toutefois, cette dernière ne peut être déterminée que si les variations de la limitation du débit sont suivies jour après jour, ambulatoirement à domicile (monitoring).

Il existe donc un besoin pour un appareil médical simple et peu coûteux permettant de mesurer le débit d'air, qui soit indépendant de toute source d'énergie électrique, facile d'emploi, tout en étant précis dans ses mesures et qui fournisse des données objectives pour l'interprétation médicale par le patient et le médecin.

La mesure ambulatoire de la limitation du débit d'air avec des appareils tels que le peak-flow meter de Wright ou le "Pulmonary Achievement Trainer", est une pratique courante chez les pédiatres, les généralistes et les spécialistes en médecine interne, lorsqu'on mesure l'obstruction des voies respiratoires chez des patients atteints de maladie pulmonaire. Le premier de ces appareils est décrit dans GB-A-1160669 et le second dans US-A-3826247. Avec de tels appareils, le débit maximum d'air qu'une personne peut expirer est mesuré dans un tube cylindrique dans lequel un piston ou une plaque est mis en action par l'air qui entre et est déplacé de sa position initiale zéro à une distance étalonnée reflétant le débit maximum d'air expiré. Pratiquement, l'utilisation de tels appareils a montré qu'une calibration en termes absolus était difficile, les variations pour un individu donné étant élevées et les comparaisons entre individus plutôt difficiles. L'application est donc limitée à une surveillance (monitoring) à long terme dans laquelle le patient est son propre contrôle. Cette application est aussi restreinte aux mesures de débit d'air maximum et, par conséquent, aux mesures du débit expiratoire de pointe (peak-flow). Dans ce contexte, il faut reconnaître que les mesures de peak-flow ne représentent que la pointe de l'iceberg, en quelque sorte, des désordres fonctionnels sous-jacents, reflétant seulement les processus dans les voies respiratoires de grand diamètre, alors que d'autres obstructions sont parfois plus importantes. De plus, l'établissement des courbes débit-volume (y compris celle pour la mesure du peak-flow) par un sujet donné exigent des manoeuvres de respiration forcée relativement peu naturelles. De nombreux travaux scientifiques ont démontré que les manoeuvres de respiration forcée répétées peuvent entraîner des bronchospasmes. De plus, spécialement chez les enfants et les personnes âgées, on rencontre des difficultés à obtenir des courbes débit-volume reproductibles avec précision, en raison de la coopération limitée de ces patients.

L'état de la technique connue à ce jour est en relation avec diverses maladies pulmonaires telles que la bronchite obstructive, l'asthme bronchique, la mucoviscidose chez les adultes et les enfants, ainsi que les broncho-pneumopathies chroniques obstructives (BPCO) chez des patients adultes, nécessitant une analyse approfondie des fonctions pulmonaires d'un patient. En demandant au patient de souffler dans un appareil diagnostique, on peut détecter l'état des fonctions pulmonaires du patient. Toutefois, jusqu'ici, il n'a été proposé aucun appareil diagnostique à la fois simple et précis destiné à une utilisation au chevet du malade, à domicile, et qui permette au médecin une lecture directe des conditions des fonctions pulmonaires du patient pendant une période de thérapie prédéterminée.

Le but de la présente invention est d'une part de remédier à ces inconvénients et d'autre part de proposer un appareil de mesure du débit/volume d'air pulmonaire expiré par une personne, facilement transportable et qui permette l'enregistrement de données concernant la respiration de cette personne.

A cet effet, l'invention concerne un appareil de mesure du débit/volume d'air pulmonaire expiré par une personne, tel que défini à la revendication 1. D'autres caractéristiques de l'invention sont énoncées dans les revendications subordonnées à la revendication 1.

Pour utiliser cet appareil, la personne doit inspirer complètement, puis expirer doucement, de manière non forcée, dans l'embout de l'appareil. On constate que la personne va automatiquement ajuster sa vitesse d'expiration pour obtenir du sifflet une note stable d'amplitude sensiblement constante, ayant pour résultat que la vitesse d'expiration reste constante tout au long du test, sauf au début et à la fin.

En se basant sur des mesures répétées à différentes vitesses d'expiration, les données de la courbe débit-volume peuvent être calculées comme on le décrira plus loin. Les mesures ne nécessitent pas de manoeuvre de respiration forcée.

Outre les avantages déjà mentionnés, cet appareil peut notamment être réalisé avec de petites dimensions, inférieures à celles du peak-flow meter de Wright ou du "Pulmonary Achievement Trainer". Il peut être tenu comme un crayon et peut fonctionner sans énergie électrique.

D'autres avantages de l'invention ressortiront de la description qui suit, donnée à titre d'exemple, et qui se réfère au dessin sur lequel:

- la figure 1 illustre quelques courbes exprimant la relation entre le débit et le volume d'air expiré, l'une des courbes ayant déjà été décrite ci-dessus, les autres étant décrites plus bas;
- la figure 2 est une vue en plan d'un mode d'exécution d'un appareil selon l'invention;
- la figure 3 est une coupe longitudinale selon la ligne A-A de la figure 2; et
- la figure 4 représente un diagramme d'un exemple de circuit utilisé dans le détecteur représenté sur les figures 2 et 3.

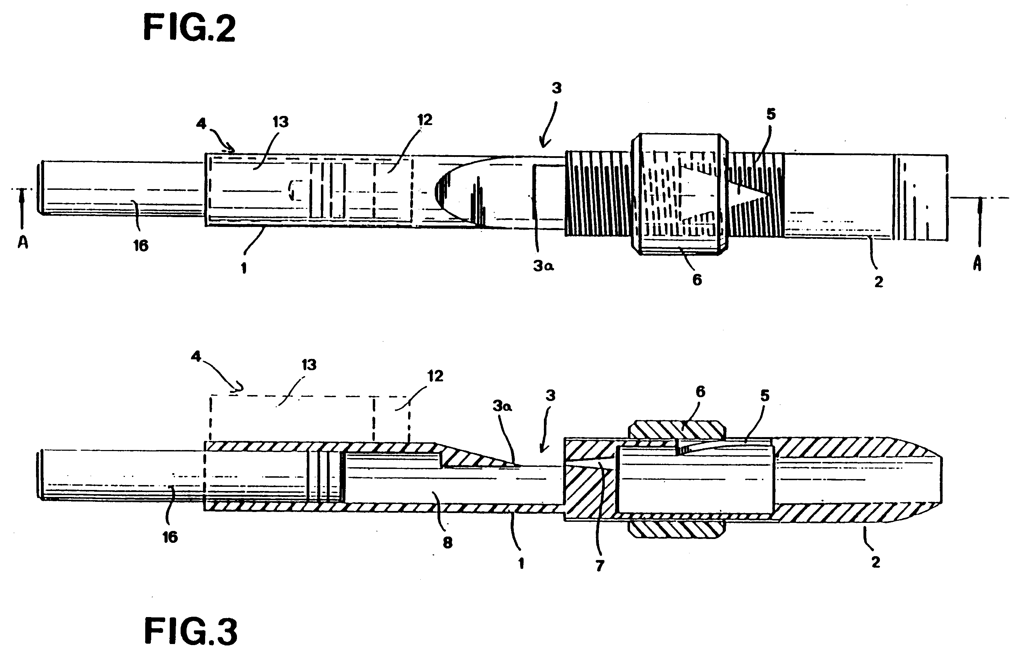

L'appareil représenté sur les figures 2 et 3 comprend un conduit de connection 1 comportant un embout 2 à l'une de ses extrémités, un générateur de son 3 muni d'un bec 3a, ainsi qu'une unité de mesure digitale du temps 4 (DTMU). Cette unité 4 n'est représenté que sous forme schématique sur les figures 2 et 3. Mis à part l'unité de mesure du temps, l'appareil peut être exécuté en matériau plastique acrylique translucide, l'unité de mesure du temps 4 étant de préférence déconnectable, de façon qu'une fois cette unité déconnectée, l'appareil puisse être nettoyée et stérilisé. Le diamètre intérieur de l'embout 2 est plus petit que le conduit de connection 1. Son orifice d'extrémité est de forme ovale afin de bien s'adapter à la bouche du patient, puis cette forme se modifie à l'approche du conduit de connection 1 pour aboutir à une section transversale circulaire. Selon une variante, spécialement destinée aux enfants, l'embout peut être remplacé par un masque facial. Le conduit de connection 1 a pour fonction de définir la quantité d'air qui est conduite à travers le générateur de son 3. L'orifice d'échappement de dérivation 5 de forme triangulaire permet la dérivation d'une partie de l'air expiré. Cet orifice 5 comporte une ouverture réglable en tournant simplement une manchette 6, vissée à l'extérieur du conduit 1. Lorsque le patient expire dans l'appareil, seule une fraction bien définie du flux d'air est dirigée vers le générateur de son 3, le reste étant éliminé dans l'atmosphère par l'échappement de dérivation 5. Le flux d'air qui reste est dirigé à travers le générateur de son 3 dans lequel une note bien définie est produite. Le conduit de connection 1 peut comporter une échelle qui peut être calibrée au moyen d'un pneumotachographe. De cette façon, le flux d'air dirigé à travers le générateur de son 3 peut être défini en termes absolus (litres/secondes).

Le générateur de son 3 est construit selon les principes classiques de l'instrument à vent qu'est la flûte à bec. Le flux d'air passe à travers un conduit 7 et percute le bec 3a, créant une résonance dans la chambre de résonance 8. La grandeur (longueur et diamètre) de la chambre de résonance 8 définit la fréquence de la note. Selon un mode d'exécution, une note d'une fréquence de 1650 Herz est choisie. Cette fréquence est précisément adaptée à la bande passante de l'unité de mesure du temps (DTMU). Des essais ont montré que la sensibilité du générateur de son 3, et par conséquent la reproductibilité des mesures de volume, dépendent de façon critique de l'angle du bec 3a. En particulier, un angle de 12 degrés permet de produire une note de bonne qualité avec un débit très faible de l'ordre de 0.1 litre/seconde. On a pu constater que, même avec un débit si faible, des variations de la vitesse du flux de ±5 % sont suffisantes pour empêcher la génération d'une note de bonne qualité. Il s'ensuit que la production d'une telle note est une indication précise qu'un débit particulier a été obtenu. En fait, les débits concernés lorsque l'appareil est utilisé par un patient sont de 0.2 litre/seconde et plus. Avec de tels débits, on peut s'attendre à une reproductibilité encore meilleure.

Afin de permettre un ajustement de la fréquence de résonance de la chambre 8 à la valeur souhaitée, cette chambre peut être munie d'un piston coulissant 16. Une fois l'ajustement réalisé, on bloque le piston. Bien entendu, tout autre dispositif permettant de faire varier les dimensions de la chambre de résonance afin d'adapter la fréquence peut être envisagé.

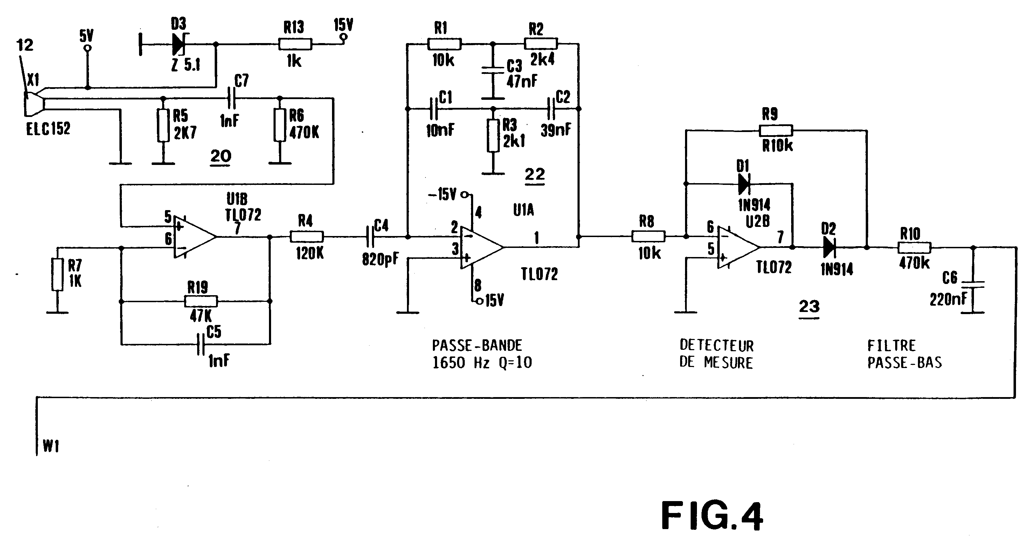

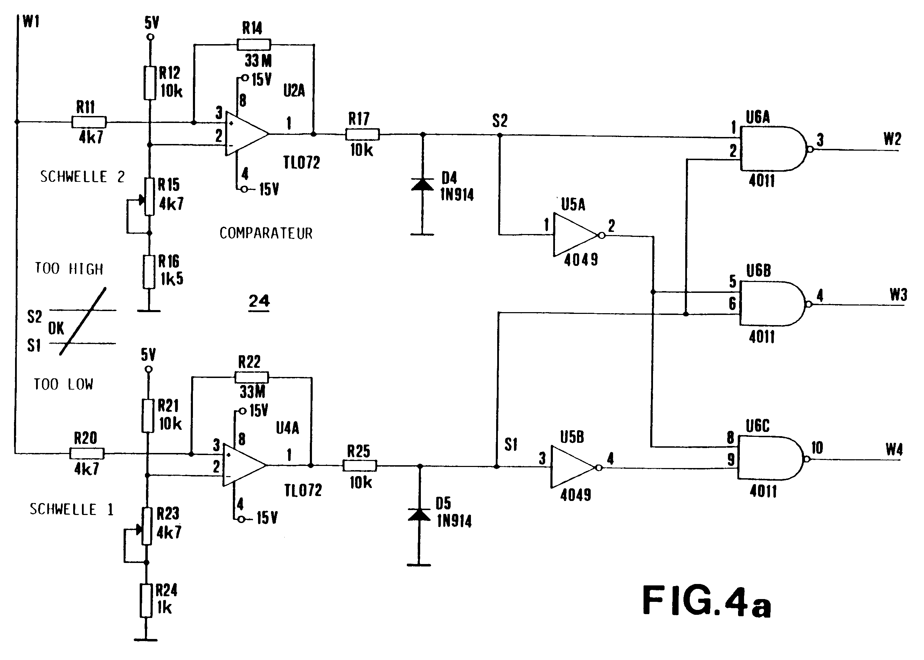

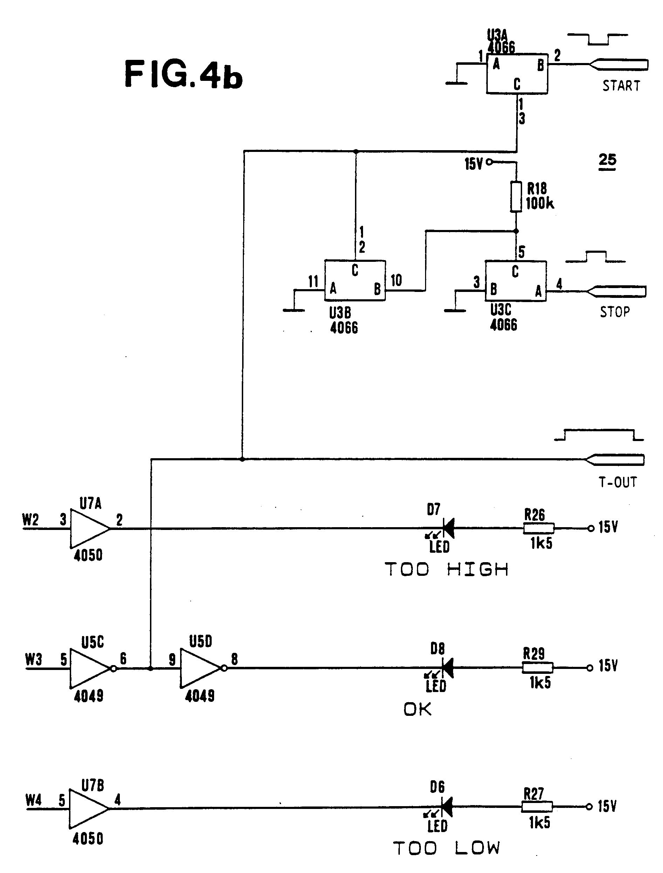

L'unité de mesure du temps (DTMU) comprend un minimicrophone 12 et un circuit électronique 13. Cette unité peut aussi être équipée d'un affichage digital (non représenté). Le microphone transforme les ondes sonores en courant alternatif. Un exemple de circuit électronique est représenté à la figure 4. Ce circuit traite le signal de sortie du microphone 12. Il comprend un filtre passe-haut 20 qui filtre, en les éliminant, toutes les fréquences inférieures à 100 Herz. Le signal filtré est alors envoyé vers un amplificateur 21 destiné à amplifier le courant alternatif d'un facteur d'environ 50, pour passer de quelques mV à des valeurs de l'ordre de 200 mV. Une fois amplifié, le signal est envoyé vers un filtre passe-bande 22, qui filtre, en les éliminant, toutes les fréquences qui ne sont pas comprises dans une bande étroite centrée à 1650 Herz, et qui amplifie encore le signal filtré. Le signal est alors transformé dans le circuit 23 en une tension numérique. Ce signal, qui est dépendant de l'intensité sonore, est comparé à une valeur seuil fixe dans deux comparateurs d'amplitude et de fréquence 24. Les comparateurs fournissent un signal de sortie (T-out) aussi longtemps que le signal numérique est supérieur au seuil. La durée d'émission à T-out est mesurée par une horloge électronique. Une façon alternative de mesurer la durée de la note consiste à démarrer et arrêter une horloge en produisant un signal positif au début et à la fin du signal détecté. Cette possibilité est aussi représentée dans le circuit 25 du schéma électronique de la figure 4. La durée d'émission de la note s'avère directement proportionnelle au volume d'air expiré et cette durée peut par conséquent être utilisée pour produire directement, à partir de ce volume, une valeur qui peut être affichée.

En ouvrant graduellement l'échappement de dérivation triangulaire 5 au moyen de la manchette tournante 6 , on peut modifier la proportion du flux d'air passant à travers le générateur de son. En effectuant des mesures répétées pour différentes positions de la manchette, on peut établir une pluralité de courbes, chacune correspondant à une vitesse d'expiration constante différente. Trois courbes de ce type sont illustrées à la figure 1. Chacune de ces courbes correspond respectivement à des débits de 1, 2 et 3 litres/seconde. L'enveloppe de ces courbes est la courbe de peakflow générée avec les dispositifs de l'art antérieur.

On notera que l'appareil selon l'invention est de construction simple et ne comporte pas de composant mobile, de sorte que son fonctionnement ne risque pas d'être perturbé par un éventuel captage de matières étrangères. Il en résulte qu'un fonctionnement fiable peut être attendu sans nettoyage continu, ce qui constitue un avantage important, puisque l'appareil est exposé au mucus et à la salive du patient.

La sensibilité et la reproductibilité des mesures ont été évaluées par l'étude de mesures répétées avec différents débits constants, produits expérimentalement et créés par des personnes expirant dans l'appareil. La reproductibilité a été calculée. Elle est de l'ordre de 70 msec.

Du point de vue simplicité et économie de fabrication, toutes les parties, à l'exception de l'unité de mesure du temps, peuvent être réalisées par moulage à partir de matériaux plastiques, ce qui réduit les besoins en usinage et autres opérations à un minimum. De plus, le mode d'exécution décrit ci-dessus peut être réalisé en plusieurs éléments séparables, ce qui permet un démontage facile pour le nettoyage.

Selon un mode d'exécution préférentiel, l'échelle sur le conduit de connection est de forme curviligne, son étendue tenant compte de l'utilisation de l'appareil à la fois par des enfants et par des adultes. Des résultats probants ont été obtenus avec un appareil tel que décrit ci-dessus ayant des dimensions extérieures d'environ 12.9 centimètres de long et 18 millimètres de diamètre intérieur, une chambre de résonance du générateur de son de 75 millimètres de long et 15 millimètres de diamètre. Comme on l'a mentionné plus haut, les dimensions de la chambre peuvent être modifiées afin de modifier la fréquence de la note, avec un changement correspondant de la bande de fréquence.

De nombreuses modifications peuvent bien entendu être apportées à l'appareil. Par exemple, le circuit électronique peut être agrandi en ajoutant une ou plusieurs mémoires afin d'étendre les possibilités de surveillance à domicile, pour mémoriser les lectures quotidiennes du matin et du soir, ainsi que les mesures avant et après médication.

En outre, le circuit électronique de l'appareil peut être agencé de façon à générer, à partir de différentes durées d'émission de note et par conséquent de différents volumes correspondant chacun à un isoflow spécifique, une pluralité de paramètres caractérisant l'expiration d'une personne et par conséquent les paramètres de la courbe débit/volume, tels que par exemple débit de pointe, volume d'expiration à 0.5, 1, 1.5, 2, 3, 4 et 5 litres par seconde, ainsi que débits d'expiration à 75%, 50% et 25% de la capacité vitale. Il suffit alors de munir l'appareil de mémoires en nombre suffisant pour mémoriser les signaux correspondant aux paramètres calculés, de façon à pouvoir afficher sur un écran dont peut être muni l'appareil un ensemble complet de tests de fonctionnement consécutifs du poumon.

L'appareil peut comporter un boîtier contenant un microprocesseur alimenté par une batterie, agencé en particulier pour la mesure de l'air expiré, et un écran disposé sur le boîtier pour afficher ces mesures. Il peut en outre comporter des moyens de connection pour sa connection à une unité périphérique de collecte de données (PBCU), de façon à communiquer par l'intermédiaire d'un interface avec un ordinateur personnel ou un autre appareil de ce type.

D'autre part, l'appareil peut comporter des moyens pour sa connection à un dispositif d'inhalation activée d'air, par l'intermédiaire duquel le patient peut respirer des médicaments à base de poudre, tels que des béta-stimulants, des stéroïdes topiques ou d'autres produits anti-asthmatiques destinés à contrôler la mise en oeuvre correcte de la thérapie.

On explique ci-après comment l'utilisation d'un sifflet permet d'atteindre un débit d'air expiré constant.

Lorsque l'échappement 5 du sifflet est complètement fermé par la manchette 6, le sifflet fonctionne exactement comme une flûte à bec. La seule différence est que seule une note particulière bien définie est produite (à une fréquence de 1650 Herz dans l'exemple décrit ci-dessus). Après une inspiration initiale totale (jusqu'à la capacité pulmonaire totale CPT), aussi longtemps que le patient expire à travers le sifflet et s'assure qu'une note constante est produite par la flûte à bec, un débit constant est maintenu. En physiologie pulmonaire, un tel processus est désigné par le nom de production d'un "isoflow". La constance de cet isoflow est assurée par le mécanisme de rétroaction physiologique entre l'oreille et les muscles volontaires nécessaires à l'expiration. L'isoflow qui est produit avec l'échappement de dérivation 5 fermé est le flux le plus bas qui puisse être mesuré avec cet appareil.

En expirant dans le sifflet à partir de la capacité pulmonaire totale (CPT) jusqu'au volume résiduel (VR) lorsque l'échappement de dérivation est ouvert à une position donnée, on produit à nouveau un son, bien qu'une partie de l'air soit dérivé par l'échappement de dérivation. Comme la fréquence du sifflet reste la même, un débit plus élevé est nécessaire pour produire la même note que précédemment. Comme auparavant, la note est en corrélation étroite avec le flux d'air qui traverse le sifflet. La relation exacte entre le flux d'air produisant une note et la position de la manchette tournante sur l'échelle du conduit de connection est définie par des mesures parallèles avec un pneumotachographe placé en série avec le sifflet. Par conséquent, pour chaque ouverture donnée de l'échappement de dérivation triangulaire, un flux d'air correspondant bien défini peut être généré. Le temps pendant lequel la note est produite est directement en relation avec le volume d'air à un débit constant spécifique. En faisant varier l'échappement de dérivation, et par conséquent l'isoflow, on peut obtenir des données relatives à la courbe débit/volume. En mesurant la durée de la note et le débit donné pour une position donnée de la manchette on peut calculer le volume expiré :

$$volume = temps\ (s)\ x\ débit\ (l/s).$$

Par ce procédé, les différents volumes en relation avec les débits correspondants peuvent être calculés.

Selon un autre mode d'exécution, l'appareil peut être agencé de façon inverse, de façon à mesurer des débits d'inspiration selon les mêmes principes.

L'utilisation de l'appareil selon l'invention n'est pas limitée à la mesure du débit/volume d'air pulmonaire expiré par une personne. En effet l'appareil ne perd aucune de ses caractéristiques fonctionnelles, ni ses avantages et son utilité s'il est appliqué à la régulation de tout flux d'air, peu importe le contexte ou l'appareil qu'il s'agit de contrôler.

**Revendications**

1. Appareil de mesure du débit/volume d'air pulmonaire expiré par une personne, caractérisé en ce qu'il comprend un générateur de son dans lequel la personne souffle, agencé de façon à générer, sous l'effet de ce souffle, un son de fréquence et d'amplitude prédéterminées, et des moyens pour mesurer la durée de ce son, l'appareil comportant un échappement de dérivation à ouverture/fermeture variable, de façon à diminuer ou augmenter la quantité d'air qui passe à travers le générateur de son, d'où il résulte qu'en effectuant des mesures pour différentes ouvertures de la dérivation, on peut calculer les paramètres de la courbe débit-volume d'expiration de la personne concernée.

2. Appareil selon la revendication 1, caractérisé en ce qu'il comporte un embout, un générateur de son muni d'un bec et une unité de mesure du temps, l'embout et le générateur de son étant reliés par un conduit de connection sur lequel est pratiqué l'échappement de dérivation à ouverture/fermeture variable.

**3.** Appareil selon la revendication 2, caractérisé en ce que l'embout est remplacé par un masque facial.

**4.** Appareil selon l'une des revendications précédentes, caractérisé en ce qu'il comporte un microphone destiné à capter le son de fréquence et amplitude prédéterminées, ledit microphone comportant des moyens pour générer un signal dans un circuit électronique comportant des moyens pour activer une unité de mesure du temps, ladite unité étant agencée pour mesurer la durée pendant laquelle le générateur de son produit ladite note et par conséquent la durée pendant laquelle le signal est généré à travers le circuit.

**5.** Appareil selon la revendication 4, caractérisé en ce qu'il comporte au moins une mémoire destinée à recevoir la valeur de ladite durée et un écran d'affichage.

**6.** Appareil selon l'une des revendications 4 ou 5, caractérisé en ce qu'il comporte une pluralité de mémoires destinées à recevoir les valeurs des durées d'une pluralité d'émissions de notes, et des moyens de rappel et de traitement de ces valeurs agencés de façon à contrôler la compliance du patient.

**7.** Appareil selon la revendication précédente, caractérisé en ce qu'il comporte des moyens pour générer, à partir de différentes durées d'émission de note et par conséquent de différents volumes correspondant chacun à un isoflow spécifique, une pluralité de paramètres caractérisant l'expiration d'une personne et par conséquent les paramètres de la courbe débit/volume: débit de pointe, volume d'expiration à 0.5, 1, 1.5, 2, 3, 4 et 5 litres par seconde, ainsi que débits d'expiration à 75%, 50% et 25% de la capacité vitale.

**8.** Appareil selon la revendication précédente, caractérisé en ce qu'il comporte en outre des moyens pour mémoriser les signaux correspondant à l'un ou plusieurs des paramètres calculés, caractérisant un ensemble complet de tests de fonctionnement consécutifs du poumon.

**9.** Appareil selon l'une des revendications précédentes, caractérisé en ce qu'il comporte des moyens pour modifier la longueur de la chambre de résonance du générateur de son, de façon à permettre la modification de la fréquence de la note générée par le générateur de son.

**10.** Appareil selon l'une des revendications précédentes, caractérisé en ce qu'il comporte un boîtier contenant un microprocesseur alimenté par une batterie, agencé pour la mesure de l'air expiré, et un écran disposé sur le boîtier pour afficher ces mesures.

**11.** Appareil selon l'une des revendications précédentes, caractérisé en ce qu'il comporte des moyens pour sa connection à une unité périphérique de collecte de données (PBCU), de façon à communiquer par l'intermédiaire d'un interface avec un ordinateur personnel ou un autre appareil de ce type.

**12.** Appareil selon l'une des revendications précédentes, caractérisé en ce qu'il est agencé de façon inverse, de façon à mesurer des débits d'inspiration selon les mêmes principes.

**13.** Appareil selon l'une des revendication précédentes, caractérisé en ce qu'il comporte des moyens pour sa connection à un dispositif d'inhalation activée d'air, par l'intermédiaire duquel le patient respire des médicaments à base de poudre tels que des béta-stimulants, des stéroïdes topiques ou d'autres produits anti-asthmatiques destinés à contrôler la mise en oeuvre correcte de la thérapie.

**14.** Utilisation de l'appareil selon l'une des revendications précédentes pour le contrôle du débit d'un flux d'air.

**Claims**

**1.** Device for measuring the flow-volume of pulmonary air breathed out by a subject, characterized in that it comprises a sound generator into which the subject blows, arranged to produce, under the effect of said blow, a sound which has a predetermined frequency and amplitude, and means for measuring the length of said sound, said device comprising a by-pass outlet having a variable opening/closing, adapted to reduce or increase the amount of air which passes through the sound generator, the result being that by making measurements for various openings of the by-pass, the parameters of the expiratory flow-volume curve of the concerned subject can be calculated.

**2.** Device according to claim 1, characterized in that it comprises a mouthpiece, a sound generator provided with a lip and a time measuring unit, the mouthpiece and the sound generator being connected by a connecting duct on which is made the by-pass outlet having a variable opening/closing.

3. Device according to claim 2, characterized in that the mouthpiece is replaced by a face mask.

4. Device according to one of the preceding claims, characterized in that it comprises a microphone intended to pick up said sound of predetermined frequency and amplitude, said microphone comprising means for producing a signal in an electric circuit comprising means for activating a time measuring unit, said unit being arranged to measure the length of time during which the sound generator produces said note and hence the length of time during which the signal is produced through the circuit.

5. Device according to claim 4, characterized in that it comprises at least one storage means intended to receive the value of said length of time and a display screen.

6. Device according to one of claims 4 or 5, characterized in that it comprises a plurality of storage means intended to receive the values of the lengths of time of a plurality of note emissions and means for recalling and processing said values and arranged for monitoring the compliance of the patient.

7. Device according to the preceding claim, characterized in that it comprises means for producing, from different note lengths and hence from different volumes each corresponding to a specific isoflow, a plurality of parameters characterizing the exhalation of a person and hence the parameters of the flow-volume curve: peak-flow, expiratory volumes at 0.5, 1, 1.5, 2, 3, 4 and 5 litres/second, as well as expiratory flows at 75%, 50% and 25% of vital capacity.

8. Device according to the preceding claim, characterized in that it further comprises means for storing the signals corresponding to one or several of the calculated parameters characterizing a complete set of consecutive lung function tests.

9. Device according to one of the preceding claims, characterized in that it comprises means for modifying the length of the resonance chamber of the sound generator, in order to permit the modification of the frequency of the note produced by the sound generator.

10. Device according to one of the preceding claims, characterized in that it comprises a housing containing a battery driven microprocessor, arranged for the measurement of the exhaled air, and a screen disposed on the housing to display said measurements.

11. Device according to one of the preceding claims, characterized in that it comprises means for its connection to a peripheral data collecting unit (PDCU), adapted to communicate via an interface with a personal computer or other like apparatus.

12. Device according to one of the preceding claims, characterized in that it is arranged inversely, in order to measure inspiration flows according to the same principles.

13. Device according to one of the preceding claims, characterized in that it comprises means for its connection to a breath actuated inhalation device, by which patient can breathe in powder drugs, such as beta-stimulants, topic steroids or other anti-asthmatic products intended to check the proper use of the therapy.

14. Use of the device according to one of the preceding claims, for the regulation of the rate of an airflow.

**Patentansprüche**

1. Vorrichtung zur Messung der Leistung/des Volumens der von einer Person ausgeatmeten Atemluft, dadurch gekennzeichnet, daß sie einen Tongenerator enthält, in den die Person bläst und der in der Lage ist, unter der Einwirkung dieses Einblasens einen Ton einer vorgegebenen Frequenz und einer vorgegebenen Amplitude zu erzeugen, sowie eine Anordnung enthält zur Messung der Tondauer und die eine Abzweigleitung mit variabler Öffnung/variablem Verschluß aufweist, um so die Menge an Luft zu verringern oder zu erhöhen, welche den Tongenerator durchsetzt, so daß aus der Durchführung von Messungen für verschiedene Öffnungen der Abzweigung die Parameter der Leistung-Volumen-Kurve der Ausatmung der Person berechnet werden können.

2. Vorrichtung nach Anspruch 1, dadurch gekennzeichnet, daß sie einen Stutzen, einen mit einer Schneide versehenen Tongenerator und eine Zeitmeßeinheit aufweist, wobei der Stutzen und der Tongenerator durch eine Verbindungsleitung miteinander verbunden sind, an der die Abzweigleitung mit variabler Öffnung/variablem Verschluß vorgesehen ist.

3. Vorrichtung nach Anspruch 2, dadurch gekennzeichnet, daß der Stutzen durch eine Gesichtsmaske ersetzt ist.

4. Vorrichtung nach einem der vorhergehenden Ansprüche, dadurch gekennzeichnet, daß sie ein Mikrophon aufweist zur Aufnahme des Tones vorgegebener Frequenz und vorgegebener Amplitude, wobei das Mikrophon eine Anordnung enthält zur Erzeugung eines Signals in einer elektronischen Schaltung, welche eine Anordnung aufweist, um eine Zeitmeßeinheit zu aktivieren, die derart ausgelegt ist, daß sie die Dauer mißt, während der der Tongenerator den Ton erzeugt, und damit die Dauer, während der das Signal vom Schaltkreis erzeugt wird.

5. Vorrichtung nach Anspruch 4, dadurch gekennzeichnet, daß sie wenigstens einen Speicher aufweist zur Aufnahme der Werte sowie einen Anzeigebildschirm aufweist.

6. Vorrichtung nach einem der Ansprüche 4 oder 5, dadurch gekennzeichnet, daß sie eine Vielzahl von Speichern aufweist zur Aufnahme der Werte der Dauer einer Vielzahl von erzeugten Tönen sowie eine Anordnung zur Abgabe und zur Verarbeitung dieser werte im Hinblick auf eine Überwachung des Gesundheitszustandes des Patienten.

7. Vorrichtung nach einem der vorhergehenden Ansprüche, dadurch gekennzeichnet, daß sie eine Anordnung aufweist zur Erzeugung einer Vielzahl von Parametern, ausgehend von unterschiedlichen Klangdauern des Tones und demzufolge unterschiedlicher Volumina, deren jedes einem speziellen Isofluß entspricht zur Kennzeichnung der Ausatmung einer Person und damit von Parametern der Kurve Leistung/Volumen: Spitzenleistung, Ausatmungsvolumen bei 0,5, 1, 1,5, 2, 3, 4 und 5 Litern pro Sekunde, sowie Ausatmungsleistungen bei 75%, 50% und 25% der vitalen Menge.

8. Vorrichtung nach dem vorhergehenden Anspruch, dadurch gekennzeichnet, daß sie außerdem eine Anordnung aufweist zur Speicherung der Signale entsprechend einem oder mehreren der berechneten Parameter, welche eine vollständige Sammlung von aufeinanderfolgenden Lungenuntersuchungen charakterisieren.

9. Vorrichtung nach einem der vorhergehenden Ansprüche, dadurch gekennzeichnet, daß sie eine Anordnung aufweist zur Veränderung der Länge der Resonanzkammer des Tongenerators, um so eine Veränderung der Frequenz des vom Tongenerator erzeugten Tones zu ermöglichen.

10. Vorrichtung nach einem der vorhergehenden Ansprüche, dadurch gekennzeichnet, daß sie ein Gehäuse aufweist, in dem ein batteriegespeister Mikroprozessor angeordnet ist zur Messung der ausgeatmeten Luft, sowie einen Bildschirm am Gehäuse zur Anzeige dieser Messungen.

11. Vorrichtung nach einem der vorhergehenden Ansprüche, dadurch gekennzeichnet, daß sie eine Anordnung aufweist, um eine Verbindung mit einer äußeren Datenaufnahmevorrichtung (PBCU) herzustellen, und um über eine Schnittstelle mit einem Personalcomputer oder einem anderen ähnlichen Datenverarbeitungsgerät zu kommunizieren.

12. Vorrichtung nach einem der vorhergehenden Ansprüche, dadurch gekennzeichnet, daß sie in entgegengesetzter Weise einsetzbar ist, zur Messung der Einatmungsleistungen gemäß den gleichen Prinzipien.

13. Vorrichtung nach einem der vorhergehenden Ansprüche, dadurch gekennzeichnet, daß sie eine Anordnung aufweist, um sie mit einer mit Luft aktivierten Inhalationseinrichtung zu verbinden, mittels derer der Patient pulverförmige Medikamente einatmet, wie z.B. Beta-Stimulantien, örtlich wirksame Steroide oder andere gegen Asthma wirksame Produkte, zur Überwachung der korrekten Durchführung der Therapie.

14. Verwendung der Vorrichtung nach einem der vorhergehenden Ansprüche für die Überwachung der Leistung eines Luftstromes.

# FIG.1

# FIG.2

# FIG.3

EP 0 527 216 B1

FIG.4

EP 0 527 216 B1

FIG.4a

EP 0 527 216 B1

# FIG.4b